# EUROPÄISCHE PATENTSCHRIFT

(11) **EP 3 843 619 B1**
(45) Veröffentlichungstag und Bekanntmachung des Hinweises auf die Patenterteilung: **26.04.2023**
(21) Anmeldenummer: 19762738.3
(22) Anmeldetag: 27.08.2019
(51) Int. Cl.: A61B 5/021, A61B 5/00, G16H 10/00

(54) **VERFAHREN UND VORRICHTUNG ZUM KORRIGIEREN EINER AN EINEM MESSORT VORGENOMMENEN BLUTDRUCKMESSUNG**
METHOD AND APPARATUS FOR CORRECTING A BLOOD PRESSURE MEASUREMENT TAKEN AT A MEASUREMENT LOCATION
PROCÉDÉ ET APPAREIL POUR CORRIGER UNE MESURE DE PRESSION ARTÉRIELLE PRISE À UN ENDROIT SPÉCIFIQUE

(30) Priorität: 29.08.2018 DE 102018006844
(43) Veröffentlichungstag der Anmeldung: 07.07.2021
(73) Patentinhaber: Pulsion Medical Systems SE, 85622 Feldkirchen (DE)
(72) Erfinder: THALMEIER, Thomas, 84405 Dorfen (DE); GRULER, Roman, 78628 Rottweil (DE)
(74) Vertreter: Kehl, Ascherl, Liebhoff & Ettmayr Patentanwälte Partnerschaft mbB
(86) Internationale Anmeldenummer: PCT/EP2019/072845
(87) Internationale Veröffentlichungsnummer: WO 2020/043724

(56) Entgegenhaltungen:
- WO-A1-2011/045138
- WO-A1-2014/114967
- WO-A1-2017/097905
- KR-A- 20160 042 191
- KR-B1- 101 832 486
- US-A- 5 778 879
- US-A1- 2008 021 584
- US-A1- 2015 279 186
- US-A1- 2018 225 889

## Beschreibung

### Technisches Gebiet

Die vorliegende Erfindung betrifft ein Verfahren zum Korrigieren einer an einem Messort vorgenommenen Blutdruckmessung, der eine geodätische Höhendifferenz zu einem durch die Lage des Herzens eines Patienten definierten Referenzort aufweist.

### Stand der Technik

Der (insbesondere arterielle) Blutdruck eines Patienten ist eine der wichtigsten Messgrößen in der Medizintechnik und die bekannte zugehörige Messtechnik, sowohl invasiv als auch nichtinvasiv, überaus vielfältig. Dies gilt vor allem für Messtechnik zur kontinuierlichen Überwachung des Blutdrucks über einen längeren Zeitraum, beispielsweise in der Intensivmedizin aber auch in der Notfallmedizin und während operativen Eingriffen.

Aus Gründen der guten Zugänglichkeit ist die Blutdruckmesseinrichtung dabei oft an Gliedmaßen des Patienten angebracht, beispielsweise ein applanationstonometrischer Sensor bei der Radialarterie am Unterarm oder ein photoplethysmographisch nach der sogn. "Vascular Unloading Technique" nach Peňáz betriebener Fingersensor. Derartige Druckmesseinrichtungen sind beispielsweise aus US 4,406,289, US 4,524,777, US 4,726,382, WO 2010/050798 A1, WO 2000/059369 A1, WO 2011/045138 A1, WO 2011/051819 A1, WO 2011/051822 A1, WO 2012/032413 A1 und WO 2017/143366 A1 bekannt.

Über eine Flüssigkeitssäule durch ein Katheter- und Schlauchsystem mit einer Arterie eines Patienten verbundene Drucksensoren bei invasiven Messungen können ebenfalls, beispielsweise per Klebestreifen oder Naht, mit einer Gliedmaße des Patienten verbunden oder etwas entfernt vom Patienten befestigt sein.

In allen genannten Fällen ändert ist der lokal am Messort gemessene Druck abhängig von der geodätischen Höhendifferenz zwischen dem Messort und dem Ort des Herzens des Patienten. So ändert sich beispielsweise ein an einem ortsfesten Finger oder Handgelenk gemessener arterieller Blutdruck, wenn der Patient von flach liegender in eine halbsitzende Position oder umgekehrt gebracht wird. Gleichermaßen ändert sich der an einem Finger oder Handgelenk gemessene arterieller Blutdruck, wenn bei ansonsten unveränderter Lage des Patienten die Hand angehoben oder abgesenkt wird.

Um entsprechende Lageänderungen bei kontinuierlichen Blutdruckmessungen zu korrigieren, so dass die Blutdrucküberwachung bzw. das blutdruckbasierte Patienten-Monitoring von Änderungen der geodätischen Höhendifferenz zwischen Messort und Patientenherz möglichst weitgehend unbeeinflusst bleiben, sind aus dem Stand der Technik Vorrichtungen bekannt, welche einen statischen Differenzdruck messen zwischen der am Messort befindlichen Blutdruckmesseinrichtung und einem Referenzortfestlegungsteil, der an einem durch die Lage des Patientenherzens definierten Referenzort befestigt, beispielsweise auf der Brust aufgeklebt, ist. So offenbart etwa EP 0 465 345 A1 einen plethysmographischer Blutdrucksensor mit Fingermanschette, wobei der hydrostatische Druckunterschied zum Herzen mittels einer Flüssigkeitssäule zwischen einem belüftetem Flüssigkeitsreservoir einer in Herznähe angebrachten Brusteinheit und einem Drucksensor im Bereich der Fingermanschette gemessen wird.

US 4,779,626 beschreibt einen Differenzdrucksensor an einer Fingermanschette mit hydraulischer Verbindung zu einem Flüssigkeitsreservoir in Herznähe, welches als nachgiebiger Beutel mit Schutzgehäuse ausgeführt ist.

WO 2006/020917 A1 offenbart ein System mit mindestens einem Blutdruck-Sensor (invasiv oder nicht-invasiv) und einem weiteren Drucksensor, der mit einer Kalibriervorrichtung verbunden ist, bei der es sich um ein Flüssigkeitsreservoir handeln kann.

US 5,957,853 beschreibt ein Höhenreferenzsystem mit einem Messdrucksensor für die Blutdruckmessung und einen Referenzdrucksensor.

Derartige Vorrichtungen müssen in der Regel vor ihrer Anwendung kalibriert werden. Bei elektronischen Messsystemen läuft dies üblicherweise so ab, dass der Referenzortfestlegungsteil vor Anbringen am Referenzort möglichst auf dieselbe geodätische Höhe gebracht und dann vom medizinischen Personal der Nullpunktabgleich, beispielsweise über eine Taste oder eine graphische Bedienoberfläche, manuell ausgelöst wird. Hierzu muss sich entweder das entsprechende Bedienelement, wie etwa ein Touchscreen, direkt neben dem Patienten befinden, oder aber es werden zwei Personen benötigt, welche zugleich den Referenzortfestlegungsteil in die zur Kalibrierung geeignete Lage bringen und den Nullpunktabgleich auslösen. Ferner können Kalibrierfehler entstehen, wenn der Referenzortfestlegungsteil zur Kalibrierung nicht mit ausreichender Sorgfalt in der richtigen Lage gehalten wird.

So ist auch aus KR 2016 0042191 A ein Höhenreferenzsystem bekannt, das manuell kalibriert werden muss. KR 101 832 486 B1 offenbart ein Messsystem für den Glukosegehalt im Blut mit automatischer Kalibrierung, wobei Nahfeldkommunikation (*Near Field Communication* NFC) zum Übermitteln eines Kalibrierwerts verwendet wird. Auch US 2015/279186 A1 beschreibt Nahfeldkommunikation im Zusammenhang mit physiologischen Messungen, wobei als typische Abstände zwischen Sender und Empfänger 20 bis 100 cm angegeben werden.

WO 2017/097905 A1 offenbart ein Verfahren und eine Vorrichtung zur hochpräzisen Positionsbestimmung eines mobilen Gerätes, wobei die Positionsmessung für einen Stift mittels einer Nahfeldkommunikationseinrichtung kalibriert werden kann, wenn der Stift an einen entsprechend eingerichteten Koordinatenursprung geführt wird.

### Darstellung der Erfindung

Angesichts der bei herkömmlichen Systemen bestehenden Kalibrierproblematik ist es Aufgabe der vorliegenden Erfindung, die Kalibrierung von Systemen zur Berücksichtigung eines geodätischen Differenzdrucks zwischen dem Messort der Blutdruckmessung und einem Referenzort in Herznähe zu vereinfachen und möglichst auch zuverlässiger zu gestalten.

Gemäß einem Aspekt der vorliegenden Erfindung wird diese Aufgabe mit einem Verfahren gemäß Anspruch 1 gelöst.

Gemäß einem weiteren Aspekt wird zur Lösung der der Erfindung zugrundeliegenden Aufgabe eine Vorrichtung gemäß Anspruch 4 bereitgestellt.

Bevorzugte Ausführungsformen der Erfindung können gemäß einem der abhängigen Ansprüche umgesetzt werden.

Die vorliegende Erfindung stellt somit insbesondere ein Verfahren zum Korrigieren einer an einem Messort vorgenommenen Blutdruckmessung, der eine geodätische Höhendifferenz zu einem durch die Lage des Herzens eines Patienten definierten Referenzort aufweist, bereit, wobei
- ein Referenzortfestlegungsteils im wesentlichen auf der geodätischen Höhe einer am Messort befindlichen Blutdruckmesseinrichtung platziert wird,
- eine Kalibriermessung zur Festlegung eines Nullpunkts für eine statischen Druckdifferenz zwischen dem Referenzort und dem Messort vorgenommen wird, während der Referenzortfestlegungsteil im wesentlichen auf der geodätischen Höhe der am Messort befindlichen Blutdruckmesseinrichtung platziert ist,
- der Referenzortfestlegungsteils am Referenzort platziert wird,
- die statischen Druckdifferenz zwischen dem Messort und dem Referenzort bestimmt wird. während der Referenzortfestlegungsteil am Referenzort platziert ist. und
- die Blutdruckmessung mittels der bestimmten statischen Druckdifferenz korrigiert wird. Dabei wird die Kalibriermessung durch ein zwischen Referenzortfestlegungsteil und Blutdruckmesseinrichtung ausgetauschtes Auslösesignal ausgelöst, wobei das Auslösesignal automatisch durch Platzieren des Referenzortfestlegungsteils in unmittelbarer Nähe der Blutdruckmesseinrichtung ausgelöst wird.

Entsprechend stellt die Erfindung auch eine Vorrichtung zum Korrigieren einer an einem Messort vorgenommenen Blutdruckmessung, der eine geodätische Höhendifferenz zu einem durch die Lage des Herzens eines Patienten definierten Referenzort aufweist, bereit, welche folgendes aufweist:
- eine Blutdruckmesseinrichtung zur Platzierung am Messort und zum Vornehmen der Blutdruckmessung,
- Differenzdruckbestimmungsmittel zum Bestimmen einer statischen Druckdifferenz zwischen einem momentanen Ort des Referenzortfestlegungsteil und einem momentanen Ort der Blutdruckmesseinrichtung,
- Kalibriermittel zum Vornehmen einer Kalibriermessung zur Festlegung eines Nullpunkts für die statische Druckdifferenz,
- Korrekturmittel zum Korrigieren der Blutdruckmessung mittels der bestimmten statischen Druckdifferenz, und
- Auslösemittel zum automatischen Auslösen der Kalibriermessung bei Platzierung des Referenzortfestlegungsteils in unmittelbarer Nähe der Blutdruckmesseinrichtung.

Im wesentlichen auf der geodätischen Höhe der am Messort befindlichen Blutdruckmesseinrichtung platziert bedeutet dabei eine geodätische Höhenabweichung von 30 Millimetern oder weniger. vorzugsweise 20 Millimetern oder weniger.

Unmittelbare Nähe bedeutet einen Abstand zwischen einem ersten definierten Punkt an der Blutdruckmesseinrichtung und einem zweiten definierten Punkt am Referenzortfestlegungsteil von maximal 30, vorzugsweise maximal 20 Millimetern.

Gemäß einer besonders vorteilhaften Ausführungsform wird das Auslösesignal induktiv, vorzugsweise mittels Nahfeldkommunikation (*Near Field Communication* NFC), und/oder magnetisch, beispielsweise unter Verwendung eines Reed-Schalters in der Blutdruckmesseinrichtung, der auf einen im Referenzortfestlegungsteil angeordneten Magneten reagiert, ausgelöst.

Zur Umsetzung der Nahfeldkommunikation kann vorteilhafterweise auf per se bekannte Technologie, beispielsweise aus dem Bereich drahtloser Bezahlsysteme oder Zugangskontrollen, zurückgegriffen werden. So kann gemäß einer bevorzugten Ausführungsform der Referenzortfestlegungsteil mit einem passiven HF-RFID-Tag ausgestattet werden, das bei Unterschreiten eines Abstandsschwellwerts von einem in die Blutdruckmesseinrichtung integrierten Lesegerät ausgelesen werden kann.

Insbesondere bei Verwendung von Nahfeldkommunikation können demnach vorteilhafterweise Identifikationsmittel zum Identifizieren einer Kodierung des Referenzortfestlegungsteils vorgesehen werden. Beispielsweise kann genanntes passives HF-RFID-Tag einen Code speichern, anhand dessen das zugehörige Lesegerät in der Blutdruckmesseinrichtung den Referenzortfestlegungsteil identifiziert. So kann sichergestellt werden, dass nur Referenzortfestlegungsteile geeigneter Spezifikation verwendet werden, und die Vorrichtung bei Anschluss eines falsch spezifizierten Referenzortfestlegungsteils eine Warnung ausgibt und/oder die Funktion sperrt.

Oft ist es aus technischen Gründen ein gewisser Abstand zwischen Messort und blutdruckmesseinrichtungsseitigen Auslösemitteln vorhanden. Bei einer Lageänderung der Blutdruckmesseinrichtung dergestalt, dass eine Ebene, die durch Messort und blutdruckmesseinrichtungsseitigen Auslösemittel verläuft, relativ zur Waagrechten gekippt wird, ergibt sich auch eine Verschiebung der geodätischen Höhe der blutdruckmesseinrichtungsseitigen Auslösemittel relativ zum Messort.

Vorzugsweise wird daher mittels geeigneter Lageänderungssensormittel eine Änderung der Lage und/oder der räumlichen Orientierung der Blutdruckmesseinrichtung relativ zur Waagrechten detektiert. Erkennen die Lageänderungssensormittel, dass der geodätische Höhenunterscheid zwischen Lageänderungssensormittel und Messort ein zulässiges Maß (d.h. einen Schwellenwert) überschreitet, kann eine Warnung oder aber ein entsprechender Korrekturwert ausgegeben werden. Lageänderungssensormittel können mittels Lagesensoren oder Beschleunigungssensoren implementiert werden, wie beispielsweise aus dem Stand der Technik für die Bestimmung der räumlichen Orientierung von Smartphone bekannt.

Besonders vorteilhaft kann die Erfindung eingesetzt werden, wenn Blutdruckmesseinrichtung einen Fingersensor aufweist, da gerade hier die geodätische Höhendifferenz zwischen Herz und Messort besonders groß sein und leicht verändert werden kann.

Insbesondere kann die Blutdruckmesseinrichtung vorteilhafterweise folgendes aufweisen:
- eine Strahlungsquelle zum Aussenden nahinfraroten Lichts in einen Finger durch eine optische Emissionsfläche,
- einen Photodetektor zum Detektieren eines durch eine optische Kollektorfläche aufgefangenen, in dem Finger nicht absorbierten Anteils des nahinfraroten Lichts,
- eine dem Fingersensor zugeordnete, mit einem Fluid füllbare Manschette zum Aufnehmen des Fingers, und
- ein Druckregelsystem zum Regeln eines Fluiddrucks in der Manschette in Abhängigkeit des detektierten nicht absorbierten Anteils des nahinfraroten Lichts.

Eine derart ausgestattete Blutdruckmesseinrichtung kann den arteriellen Blutdruck im Finger nach der sogenannten "Vascular Unloading Technique" bestimmen.

Vorzugsweise weist die Blutdruckmesseinrichtung einen Basisteil und einen mit dem Basisteil werkzeugfrei verbindbaren und vom Basisteil werkzeugfrei trennbaren, die Manschette umfassenden Manschettenteil auf, wobei die Auslösemittel zumindest teilweise im Basisteil angeordnet sind. Durch die Trennung von Basis- und Manschettenteil kann der hauptsächlich mit dem Patienten in Berührung stehende Manschettenteil hygienisch als Einwegartikel ausgeführt werden, während die aufwendige Mess- und Druckregeltechnik überwiegend im wiederverwertbaren Basisteil untergebracht werden kann. Auch der Referenzortfestlegungsteil kann vorteilhaft als Einwegartikel ausgeführt werden.

Gemäß einer bevorzugten Ausführungsform weist die Vorrichtung Benachrichtigungsmittel zur visuellen und/oder akustischen Benachrichtigung eines Nutzers auf, dass eine Kalibriermessung erfolgt ist. Beispielsweise mittels einer an der Blutdruckmesseinrichtung angeordneten Leuchtdiode, eines in der Blutdruckmesseinrichtung untergebrachten Tongenerators oder über ein an eine andere Ausgabevorrichtung, beispielsweise das Display eines mit der Blutdruckmesseinrichtung verbundenen Patientenmonitors, kann sich medizinisches Personal so versichern, dass das System erfolgreich kalibriert wurde. Der die Kalibrierung initiierende Benutzer kann so insbesondere feststellen, dass bzw. wann der Referenzortfestlegungsteil nahe genug zum automatischen Auslösen der Kalibriermessung an die Blutdruckmesseinrichtung herangeführt wurde.

Gemäß einer bevorzugten Ausführungsform weist die Vorrichtung Schaltmittel zum Aktivieren und Deaktivieren der Auslösemittel auf. Die Vorrichtung kann so gewissermaßen vor Benutzung "scharfgestellt" werden.

Grundsätzlich kann jede im Rahmen der vorliegenden Anmeldung beschriebene bzw. angedeutete Variante der Erfindung besonders vorteilhaft sein, je nach wirtschaftlichen, technischen und ggf. medizinischen Bedingungen im Einzelfall.

Insbesondere kann die zur Bestimmung der statischen Druckdifferenz zwischen dem Messort und dem Referenzort verwendete Technologie grundsätzlich ausgeführt sein wie aus dem Stand der Technik bekannt.

Die Erfindung wird nachfolgend beispielhaft anhand der beigefügten schematischen Zeichnungen näher erläutert. Die Zeichnungen sind nicht maßstabsgetreu; insbesondere entsprechen Verhältnisse der einzelnen Abmessungen zueinander aus Gründen der Anschaulichkeit nicht unbedingt den Abmessungsverhältnissen in tatsächlichen technischen Umsetzungen. Einander entsprechende Elemente sind in den einzelnen Figuren mit denselben Bezugszeichen gekennzeichnet.

### Kurze Beschreibung der Figuren

- Figur 1: zeigt schematisch die Gesamtanordnung einer erfindungsgemäßen Vorrichtung, wobei die Lage des Referenzortfestlegungsteils einmal in Messkonfiguration (durchgezogene Linie) und einmal in Kalibrierkonfiguration (gestrichelte Linie) dargestellt ist.
- Figur 2: zeigt schematisch eine weitere erfindungsgemäße Vorrichtung ähnlich der in Fig. 1 dargestellten in Kalibrierkonfiguration.
- Figur 3a: zeigt die Druckmesseinrichtung der Vorrichtung aus Fig. 2 auf einer waagrechten Auflage.
- Figur 3b: zeigt die Druckmesseinrichtung der Vorrichtung aus Fig. 2 in gekipptem Zustand.
- Figur 4a: zeigt die Druckmesseinrichtung wie in Fig. 3a in der Seitenansicht.
- Figur 4b: zeigt die Druckmesseinrichtung aus Fig. 4a in der Vorderansicht (von links in Fig. 4a).
- Figur 5: zeigt eine vergrößerte Ansicht der Fig. 4b mit schematisch skizzierten photoplethysmographischen Komponenten,

### Detaillierte Beschreibung vorteilhafter Ausführungsbeispiele

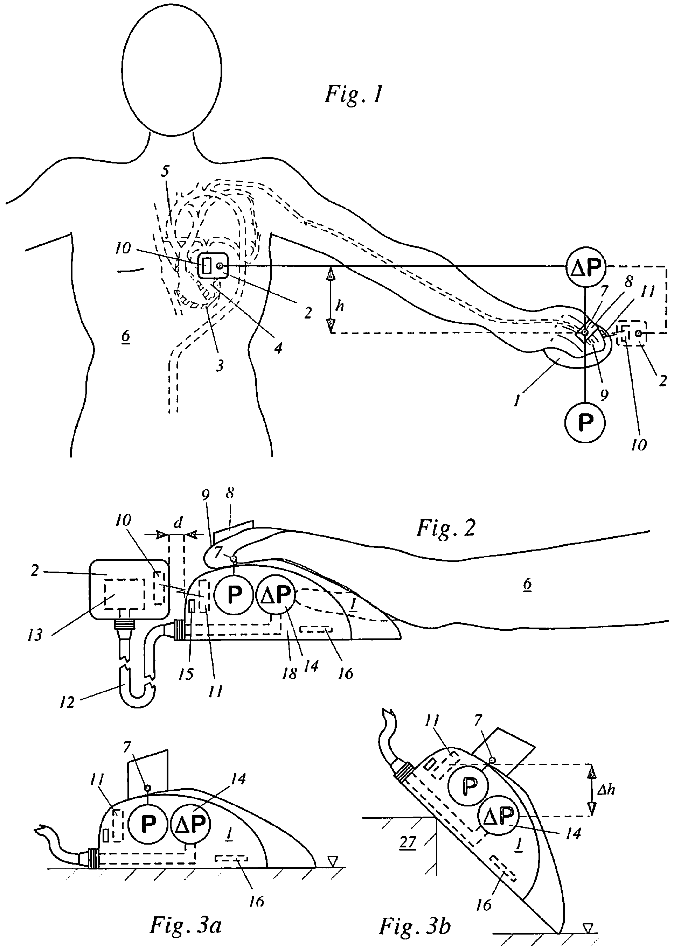

Die in Fig. 1 dargestellte Vorrichtung weist eine Blutdruckmesseinrichtung 1 und einen Referenzortfestlegungsteil 2 auf, der in Messkonfiguration nahe des Herzens 3, dabei möglichst nahe der als Referenzort anzusehenden linken Herzkammer 4 oder Aorta 5 des Patienten 6 angebracht, beispielsweise auf die Haut des Patienten 6 aufgeklebt ist. Wie grundsätzlich aus dem Stand der Technik bekannt, beispielsweise mittels einer Flüssigkeitssäule zwischen Blutdruckmesseinrichtung 1 und Referenzortfestlegungsteil 2 mit blutdruckmesseinrichtungsseitigem Drucksensor oder an beiden Enden der Flüssigkeitssäule angeordneten Drucksensoren, misst die Vorrichtung einen aus dem geodätischen Höhenunterschied h zwischen Referenzortfestlegungsteil 2 und nahe dem Messort befindlicher Blutdruckmesseinrichtung 1 resultierenden Druckunterschied ΔP, um hiermit den von der Blutdruckmesseinrichtung 1 gemessenen Blutdruck P zu korrigieren.

Im dargestellten Beispiel liegt der Messort 7 an einem von einem Manschettenteil 8 umfassten Finger 9 des Patienten 6.

Zum Kalibrieren der Differenzdruckmessung auf einen geodätischen Höhenunterschied von (näherungsweise) null wird zuvor der Referenzortfestlegungsteil 2 so nahe an die Blutdruckmesseinrichtung 1 herangeführt, dass bei Unterschreiten eines Mindestabstands zwischen zwei jeweiligen an Referenzortfestlegungsteil 2 und Blutdruckmesseinrichtung 1 definierten Punkten ein Kalibriersignal ausgelöst wird. Die Punkte können vorteilhafterweise auf den jeweiligen Gehäusen des Referenzortfestlegungsteils 2 und der Blutdruckmesseinrichtung 1 markiert sein, so dass das Bedienpersonal einfach angewiesen werden kann, die markierten Punkte zur Kalibrierung einander anzunähern, z.B. auf einen Abstand von höchstens 3 cm, höchstens 2 cm oder höchstens 1 cm als Schwellenwert, ab welchem die Auslösemittel mit Ausgabe eines Auslösesignals reagieren.

Eine in die Blutdruckmesseinrichtung 1 integrierte oder an diese angeschlossene Steuervorrichtung (nicht dargestellt), welche vorzugsweise einen Mikroprozessor oder Mikrocontroller aufweist, nimmt die Kalibriermessung als Reaktion auf das Auslösesignal vor.

Wie in Fig. 2 illustriert, sind die Auslösemittel im beschriebenen Beispiel als referenzortfestlegungsteilseitiges RFID-Tag 10 und zugehöriges blutdruckmesseinrichtungsseitiges Nahfeldkommunikationslesegerät 11 umgesetzt. Erkennt das Nahfeldkommunikationslesegerät 11 in der Blutdruckmesseinrichtung 1 das korrekt kodierte RFID-Tag im Referenzortfestlegungsteil 2, löst es das Kalibriersignal aus. Die erfolgreiche Auslösung des Kalibriersignals und/oder der Abschluss der Kalibriermessung wird dem Benutzer durch die Leuchtdiodeneinheit (LED-Einheit) 15 angezeigt.

Vorzugsweise ist der am Patienten anzubringende Referenzortfestlegungsteil 2 mit damit einhergehenden erhöhten Anforderungen an die Sterilität als Einweg-Artikel (Wegwerfteil) ausgeführt. Er wird über die Schlauchverbindung 12 (in Fig. 2 unterbrochen dargestellt) an die Blutdruckmesseinrichtung angeschlossen, so dass eine zusammenhängende Flüssigkeitssäule zwischen einem Flüssigkeitsreservoir 13 im Referenzortfestlegungsteil 2 und einem Korrektur-Drucksensor 14 in der Blutdruckmesseinrichtung 1 besteht. Bei dem Korrektur-Drucksensor kann es sich um einen handelsüblichen, z.B. piezoelektrischen, piezoresistiven, induktiven oder kapazitiven, Drucksensor handeln.

Wie in Figuren 3a und 3b verdeutlicht, können sich Abweichungen ergeben, wenn die Blutdruckmesseinrichtung 1 gekippt wird, beispielsweise indem sie teilweise auf einem erhöhten Gegenstand 27 aufliegt. Im dargestellten Beispiel ergibt sich durch das Kippen in Verbindung mit dem Abstand zwischen Nahfeldkommunikationslesegerät 11 und Korrektur-Drucksensor 14 ein, wenn in gekippter Lage kalibriert wird, die Messkorrektur verfälschender geodätischer Höhenunterschied Δh. Je nach Einbauverhältnissen kann sich durch Kippen während der Blutdruckmessung auch ein veränderter geodätischer Höhenunterschied zwischen dem Messort 7 der Blutdruckmessung und dem Korrektur-Drucksensor 14 ergeben. Um derartige Verfälschungen arithmetisch zu korrigieren, oder bei Überschreiten eines Grenzkippwinkels relativ zur Waagrechten ein Warnsignal auszugeben, können ein oder mehrere Lage- oder Lageänderungssensoren 16 in der Blutdruckmesseinrichtung 1 vorgesehen sein.

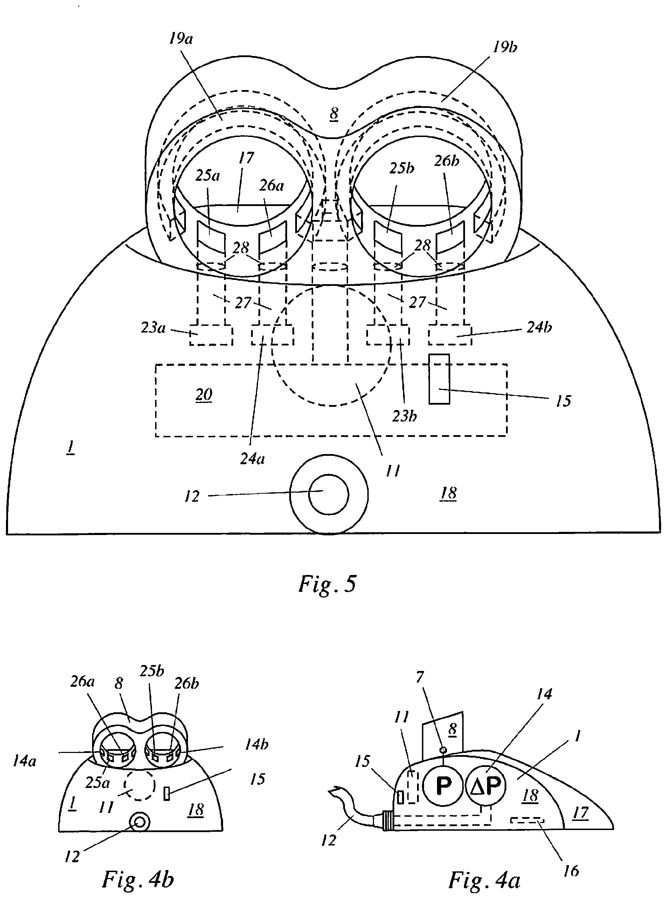

Die Blutdruckmesseinrichtung 1 selbst ist im dargestellten Beispiel als photoplethysmographisches Messsystem ausgeführt, das entsprechend der sogenannten "Vascular Unloading Technique" funktioniert. Messtechnische Komponenten können dabei grundsätzlich ähnlich wie im eingangs genannten Stand der Technik implementiert werden. Wesentliche Komponenten des beschriebenen Ausführungsbeispiels sind in Fig. 4b und vor allem Fig. 5 skizziert, welche die in Figuren 3a und 4a in Seitenansicht dargestellte Druckmesseinrichtung 1 in der Vorderansicht (von links in Figuren 3a und 4a) zeigen. Innerhalb des Gehäuses angeordnete Elemente sind strichliert angedeutet.

Der Manschettenteil 8 ist zur Aufnahme von zwei Fingern gestaltet, was wechselweises Messen an beiden Fingern möglich macht. Aus hygienischen Gründen ist der Manschettenteil 8 zusammen mit der Handflächenauflage 17 als Einwegartikel ausgeführt, der mittels einer Steckverbindung werkzeugfrei lösbar am wiederverwendbaren Basisteil 18 angebracht ist.

Die beiden aufblasbaren Fingermanschetten 19a, 19b sind über einen Verteiler 21 und einen Anschluss 22 an der Schnittstelle zwischen Manschettenteil 8 und Basisteil 18 mit dem Druckerzeugungs- und Druckregelsystem 20 verbunden. In alternativen Ausführungsformen können die Fingermanschetten 19a, 19b auch separat mit einem (optional auch jeweiligen) Druckerzeugungs- und Druckregelsystem 20 verbunden und so separat ansteuerbar sein.

Für jeden der beiden Finger ist eine beispielsweise als Leuchtdiode ausgeführte Lichtquelle 23a, 23b für nahinfrarotes Licht und ein Photodetektor 24a, 24b vorgesehen, welche über eine jeweilige sogenannte Lightpipe 27, d.h. einen nicht als Faserbündel ausgeführten Lichtleiter, mit einer zugehörigen optischen Emissionsfläche 25a, 25b bzw. optischen Kollektorfläche 26a, 26b zum Einkoppeln emittierten Lichts in das Fingergewebe bzw. Auskoppeln nicht absorbierten Lichts aus dem Fingergewebe verbunden sind. An der Schnittstelle zwischen Manschettenteil 8 und Basisteil 18 sind die manschettenteilseitigen und basisteilseitigen Abschnitte der Lightpipes 27 über trennbare optische Kontaktstellen 28 miteinander verbunden.

Das Druckerzeugungs- und Druckregelsystem 20 regelt den Manschettendruck entsprechend dem von einem der Photodetektoren 24a, 24b empfangenen Signal so, dass der nicht im entsprechenden Finger absorbierte Anteil des von der zugehörigen Lichtquelle 23a, 23b emittierten nahinfraroten Lichts möglichst konstant bleibt, d.h. es wird ein entsprechend dem pulsatilen Anteil des arteriellen Blutdrucks variierender Gegendruck erzeugt, so dass das im jeweiligen Fingerbereich vorhandene (und vom jeweiligen Lichtquellen-Detektorpaar 23a, 24a bzw. 23b, 24b plethysmographisch detektierte) Blutvolumen näherungsweise konstant bleibt. Der entsprechend vom Druckerzeugungs- und Druckregelsystem 20 geregelte Gegendruck in den Manschetten 19a, 19b wird als Blutdruckmesssignal von einem Sensor im Druckerzeugungs- und Druckregelsystem 20 erfasst und mit der mittels des Korrektur-Drucksensors 14 gemessenen geodätisch bedingte Druckdifferenz zum Referenzort korrigiert. Der korrigierte Wert kann über eine geeignete elektronische Schnittstelle an einen Patientenmonitor ausgegeben werden.

## Patentansprüche

1. Verfahren zum Korrigieren einer an einem Messort vorgenommenen Blutdruckmessung, der eine geodätische Höhendifferenz zu einem durch die Lage des Herzens (3) eines Patienten (6) definierten Referenzort aufweist, wobei das Verfahren folgendes aufweist:
Platzieren eines Referenzortfestlegungsteils (2) mit einer geodätischen Höhenabweichung von 30 Millimetern oder weniger auf der geodätischen Höhe einer am Messort (7) befindlichen Blutdruckmesseinrichtung (1),
Vornehmen einer Kalibriermessung zur Festlegung eines Nullpunkts für eine statischen Druckdifferenz zwischen dem Referenzort und dem Messort (7), während der Referenzortfestlegungsteil (2) im wesentlichen auf der geodätischen Höhe der am Messort (7) befindlichen Blutdruckmesseinrichtung (1) platziert ist,
Platzieren des Referenzortfestlegungsteils (2) am Referenzort,
Bestimmen der statischen Druckdifferenz zwischen dem Messort (7) und dem Referenzort, während der Referenzortfestlegungsteil (2) am Referenzort platziert ist,
Korrigieren der Blutdruckmessung mittels der bestimmten statischen Druckdifferenz,
**dadurch gekennzeichnet,**
**dass** die Kalibriermessung durch ein zwischen Referenzortfestlegungsteil (2) und Blutdruckmesseinrichtung (1) ausgetauschtes Auslösesignal ausgelöst wird,
wobei das Auslösesignal automatisch bei Unterschreiten eines Mindestabstands von maximal 30 Millimetern zwischen einem ersten definierten Punkt an der Blutdruckmesseinrichtung (1) und einem zweiten definierten Punkt (2) am Referenzortfestlegungsteil ausgelöst wird.

2. Verfahren gemäß Anspruch 1, wobei das Auslösesignal induktiv, vorzugsweise mittels Nahfeldkommunikation, und/oder magnetisch ausgelöst wird.

3. Verfahren gemäß einem der vorangehenden Ansprüche, wobei eine Änderung der Lage und/oder der räumlichen Orientierung der Blutdruckmesseinrichtung (1) relativ zur Waagrechten detektiert wird.

4. Vorrichtung zum Korrigieren einer an einem Messort (7) vorgenommenen Blutdruckmessung, der eine geodätische Höhendifferenz zu einem durch die Lage des Herzens (3) eines Patienten (6) definierten Referenzort aufweist, wobei die Vorrichtung folgendes aufweist:
einen Referenzortfestlegungsteil (2) zur Befestigung am Referenzort,
eine Blutdruckmesseinrichtung (1) zur Platzierung am Messort (7) und zum Vornehmen der Blutdruckmessung,
Differenzdruckbestimmungsmittel (12, 13, 14) zum Bestimmen einer statischen Druckdifferenz zwischen einem momentanen Ort des Referenzortfestlegungsteil und einem momentanen Ort der Blutdruckmesseinrichtung (1),
Kalibriermittel zum Vornehmen einer Kalibriermessung zur Festlegung eines Nullpunkts für die statische Druckdifferenz, und
Korrekturmittel zum Korrigieren der Blutdruckmessung mittels der bestimmten statischen Druckdifferenz,
**dadurch gekennzeichnet,**
**dass** die Vorrichtung Auslösemittel zum automatischen Auslösen der Kalibriermessung bei Unterschreiten eines Mindestabstands von maximal 30 Millimetern zwischen einem ersten definierten Punkt an der Blutdruckmesseinrichtung (1) und einem zweiten definierten Punkt am Referenzortfestlegungsteil (2) aufweist.

5. Vorrichtung gemäß Anspruch 4, wobei die Auslösemittel Mittel zum Erfassen elektromagnetischer Induktion zwischen im Referenzortfestlegungsteil angeordneten ersten Induktionsmitteln und in der Blutdruckmesseinrichtung angeordneten zweiten Induktionsmitteln, und/oder Mittel zum Erfassen magnetischer Kräfte zwischen ersten magnetischen Mitteln in der Blutdruckmesseinrichtung und zweiten magnetischen Mitteln im Referenzortfestlegungsteil aufweisen.

6. Vorrichtung gemäß Anspruch 5, wobei die Auslösemittel Nahfeldkommunikationsmittel (10, 11) zum Durchführen von Nahfeldkommunikation zwischen Referenzortfestlegungsteil (2) und Blutdruckmesseinrichtung (1) aufweisen.

7. Vorrichtung gemäß Anspruch 6, wobei die Nahfeldkommunikationsmittel Identifikationsmittel zum Identifizieren einer Kodierung des Referenzortfestlegungsteils (2) aufweisen.

8. Vorrichtung gemäß einem der Ansprüche 4-7, wobei die Blutdruckmesseinrichtung (1) Lageänderungssensormittel (16) zum Erfassen einer Änderung der Lage und/oder der räumlichen Orientierung der Blutdruckmesseinrichtung (1) relativ zur Waagrechten aufweist.

9. Vorrichtung gemäß einem der Ansprüche 4-8, wobei die Blutdruckmesseinrichtung einen Fingersensor aufweist.

10. Vorrichtung gemäß Anspruch 9, wobei die Blutdruckmesseinrichtung folgendes aufweist:
eine Strahlungsquelle (23a, 23b) zum Aussenden nahinfraroten Lichts in einen Finger durch eine optische Emissionsfläche (25a, 25b),
einen Photodetektor (24a, 24b) zum Detektieren eines durch eine optische Kollektorfläche (26a, 26b) aufgefangenen, in dem Finger nicht absorbierten Anteils des nahinfraroten Lichts,
eine dem Fingersensor zugeordnete, mit einem Fluid füllbare Manschette (19a, 19b) zum Aufnehmen des Fingers, und
ein Druckregelsystem (20) zum Regeln eines Fluiddrucks in der Manschette (19a, 19b) in Abhängigkeit des detektierten nicht absorbierten Anteils des nahinfraroten Lichts.

11. Vorrichtung gemäß Anspruch 10, wobei die Blutdruckmesseinrichtung einen Basisteil (18) und einen mit dem Basisteil (18) werkzeugfrei verbindbaren und vom Basisteil (18) werkzeugfrei trennbaren, die Manschette (19a, 19b) umfassenden Manschettenteil (8) aufweist, und die Auslösemittel zumindest teilweise im Basisteil (18) angeordnet sind.

12. Vorrichtung gemäß einem der Ansprüche 4-11, ferner aufweisend Benachrichtigungsmittel (15) zur visuellen und/oder akustischen Benachrichtigung eines Nutzers, dass eine Kalibriermessung erfolgt ist.

13. Vorrichtung gemäß einem der Ansprüche 4-12, ferner aufweisend Schaltmittel zum Aktivieren und Deaktivieren der Auslösemittel.

## Claims

1. A method of correcting a blood pressure measurement conducted at a measuring position having a geodetic height difference from a reference position defined by the position of a patient's (6) heart (3), the method comprising:
placing a reference position determination part (2) with a deviation of in geodetic height of 30 millimeters or less at the geodetic height of a blood pressure measuring device (1) located at the measuring position (7),
conducting a calibration measurement to establish a zero point for a static pressure difference between the reference position and the measuring position (7) while the reference position determination part (2) is positioned substantially at the geodetic height of the blood pressure measuring device (1) located at the measuring position (7),
placing the reference position determination part (2) at the reference position,
determining the static pressure difference between the measuring position (7) and the reference position while the reference position determination part (2) is placed at the reference position,
correcting the blood pressure measurement using the determined static pressure difference,
**characterized in that**
the calibration measurement is triggered by a trigger signal exchanged between the reference position determination part (2) and the blood pressure measuring device (1),
wherein the trigger signal is automatically triggered when the distance between a first defined point on the blood pressure measuring device (1) and a second defined point (2) on the reference position determination part falls below a minimum distance of not more than 30 millimeters.

2. The method according to claim 1, wherein the trigger signal is triggered inductively, preferably by means of near-field communication, and/or is triggered magnetically.

3. The method according to any one of the preceding claims, wherein a change in the position and/or the spatial orientation of the blood pressure measuring device (1) is detected relative to the horizontal.

4. A device for correcting a blood pressure measurement conducted at a measuring position (7) having a geodetic height difference from a reference position defined by the position of a patient's (6) heart (3), the device comprising:
a reference position determination part (2) for attachment to the reference position,
a blood pressure measuring device (1) for placement at the measuring position (7) and for conducting blood pressure measurements,
differential pressure determination means (12, 13, 14) for determining a static pressure difference between a current position of the reference position determination part and a current position of the blood pressure measuring device (1),
calibration means for conducting a calibration measurement to establish a zero point for the static pressure difference, and
correcting means for correcting the blood pressure measurement using the determined static pressure difference,
**characterized in that**
the device has triggering means for automatically triggering the calibration measurement when the distance between a first defined point on the blood pressure measuring device (1) and a second defined point (2) on the reference position determination part falls below a minimum distance of not more than 30 millimeters.

5. The device according to claim 4, wherein the triggering means comprise means for detecting electromagnetic induction between first induction means arranged in the reference position determination part and second induction means arranged in the blood pressure measuring device, and/or means for detecting magnetic forces between first magnetic means in the blood pressure measuring device and second magnetic means in the reference position determination part.

6. The device according to claim 5, wherein the triggering means comprise near-field communication means (10,11) for performing near-field communication between the reference position determination part (2) and the blood pressure measuring device (1).

7. The device according to claim 6, wherein the near field communication means comprises identification means for identifying a code of the reference position determination part (2).

8. The device according to any one of claims 4-7, wherein the blood pressure measuring device (1) comprises position change sensor means for detecting a change in position and/or spatial orientation of the blood pressure measuring device (1) relative to the horizontal.

9. The device according to any one of claims 4-8, wherein the blood pressure measuring device has a finger sensor.

10. The device according to claim 9, wherein the blood pressure measuring device comprises:
a radiation source (23a, 23b) for emitting near-infrared light into a finger through an optical emission surface (25a, 25b),
a photodetector (24a, 24b) for detecting a portion of the near-infrared light captured by an optical collector surface (26a, 26b) and not absorbed in the finger,
a cuff (19a, 19b) for receiving the finger, which cuff (19a, 19b) is assigned to the finger sensor and can be filled with a fluid, and
a pressure regulating system (2) for regulating a fluid pressure in the cuff (19a, 19b) as a function of the detected non-absorbed portion of the near-infrared light.

11. The device according to claim 10, wherein the blood pressure measuring device has a base part (18) and a cuff part (8) comprising the cuff (19a, 19b), which cuff part (8) can be connected to the base part (18) without tools and can be separated from the base part (18) without tools, and wherein the triggering means are at least partially arranged in the base part (18).

12. The device according to any one of claims 4-11, further comprising notification means (15) for visual and/or acoustic notification of a user that a calibration measurement has taken place.

13. The device according to any one of claims 4-12, further comprising switching means for activating and deactivating the triggering means.

## Revendications

1. Procédé de correction d'une mesure de pression artérielle prise à un endroit de mesure spécifique, qui présente une différence de hauteur géodésique par rapport à un endroit de référence défini par la position du coeur (3) d'un patient (6), dans lequel ce procédé comprend ce qui suit :
positionnement d'un élément de définition d'un endroit de référence (2) avec un écart de hauteur géodésique de 30 millimètres ou moins sur la hauteur géodésique d'un dispositif de mesure de pression artérielle (1) se trouvant à l'endroit de mesure (7),
réalisation d'une mesure d'étalonnage afin de définir un point zéro pour une différence de pression statique entre l'endroit de référence et l'endroit de mesure (7), tandis que l'élément de définition d'endroit de référence (2) est positionné globalement à la hauteur géodésique du dispositif de mesure de pression artérielle (1) se trouvant à l'endroit de mesure (7),
positionnement de l'élément de définition d'endroit de référence (2) à l'endroit de référence,
détermination de la différence de pression statique entre l'endroit de mesure (7) et l'endroit de référence, tandis que l'élément de définition d'endroit de référence (2) est positionné à l'endroit de référence,
correction de la mesure de pression artérielle au moyen de la différence de pression statique déterminée,
**caractérisé en ce que**
la mesure d'étalonnage est déclenchée par un signal de déclenchement échangé entre l'élément de définition d'endroit de référence (2) et le dispositif de mesure de pression artérielle (1),
dans lequel le signal de déclenchement est déclenché automatiquement lors du passage en dessous d'une distance minimale de maximum 30 millimètres entre le premier point défini sur le dispositif de mesure de pression artérielle (1) et un deuxième point défini sur l'élément de définition de l'endroit de référence (2).

2. Procédé selon la revendication 1, dans lequel le signal de déclenchement est déclenché de manière inductive, de préférence au moyen d'une communication à courte portée, et/ou de manière magnétique.

3. Procédé selon l'une des revendications précédentes, dans lequel une variation de la position et/ou de l'orientation dans l'espace du dispositif de mesure de la pression artérielle (1) par rapport à l'horizontale est détectée.

4. Dispositif de correction d'une mesure de pression artérielle prise à un endroit de mesure (7), qui présente une différence de hauteur géodésique par rapport à un endroit de référence défini par la position du coeur (3) d'un patient (6), dans lequel ce dispositif comprend ce qui suit :
un élément de définition d'un endroit de référence (2) destiné à être fixé à l'endroit de référence,
un dispositif de mesure de pression artérielle (1) destiné à être positionné à l'endroit de mesure (7) et pour la réalisation de la mesure de la pression artérielle,
des moyens de détermination de pression différentielle (12, 13, 14) pour la détermination d'une différence de pression statique entre l'endroit instantané de l'élément de définition de l'endroit de référence et un endroit instantané du dispositif de mesure de pression artérielle (1),
des moyens d'étalonnage pour la réalisation d'une mesure d'étalonnage afin de définir un point zéro pour la différence de pression statique et
des moyens de correction pour la correction de la mesure de pression artérielle au moyen de la différence de pression statique,
**caractérisé en ce que**
le dispositif comprend des moyens de déclenchement pour le déclenchement automatique de la mesure d'étalonnage lors d'un passage en dessous d'une distance minimale de maximum 30 millimètres entre un premier point défini sur le dispositif de mesure de pression artérielle (1) et un deuxième point défini sur l'élément de définition de l'endroit de référence (2).

5. Dispositif selon la revendication 4, dans lequel les moyens de déclenchement comprennent des moyens pour la mesure d'une induction électromagnétique entre des premiers moyens d'induction disposés dans l'élément de définition de l'endroit de référence et des deuxièmes moyens d'induction disposés dans le dispositif de mesure de pression artérielle et/ou des moyens pour la mesure de forces magnétiques entre des premiers moyens magnétiques dans le dispositif de mesure de pression artérielle et des deuxièmes moyens magnétiques dans l'élément de définition de l'endroit de référence.

6. Dispositif selon la revendication 5, dans lequel les moyens de déclenchement comprennent des moyens de communication à courte portée (10, 11) pour l'établissement d'une communication à courte portée entre l'élément de définition de l'endroit de référence (2) et le dispositif de mesure de pression artérielle (1).

7. Dispositif selon la revendication 6, dans lequel les moyens de communication à courte portée comprennent des moyens d'identification pour l'identification d'un codage de l'élément de définition de l'endroit de référence (2).

8. Dispositif selon l'une des revendications 4 à 7, dans lequel le dispositif de mesure de pression artérielle (1) comprend des moyens de capteurs de variation de position (16) pour la mesure d'une variation de la position et/ou de l'orientation dans l'espace du dispositif de mesure de pression artérielle (1) par rapport à l'horizontale.

9. Dispositif selon l'une des revendications 4 à 8, dans lequel le dispositif de mesure de pression artérielle comprend un capteur de doigt.

10. Dispositif selon la revendication 9, dans lequel le dispositif de mesure de pression artérielle comprend ce qui suit :
une source de rayonnement (23a, 23b) pour l'émission d'une lumière infrarouge proche dans un doigt par l'intermédiaire d'une surface d'émission optique (25a, 25b),
un photodétecteur (24a, 24b) pour la détection d'une part non absorbée dans le doigt de la lumière infrarouge proche, capturée par une surface collectrice optique (26a, 26b),
une manchette (19a, 19b), correspondant au capteur de doigt et pouvant être remplie d'un fluide, pour le logement du doigt et
un système de régulation de pression (20) pour la régulation d'une pression du fluide dans la manchette (19a, 19b) en fonction de la part non absorbée et détectée de la lumière infrarouge proche.

11. Dispositif selon la revendication 10, dans lequel le dispositif de mesure de pression artérielle comprend une partie de base (18) et une partie de manchette (8) pouvant être reliée sans outil avec la partie de base (18) et séparée sans outils de la partie de base (18), comprenant la manchette (19a, 19b), et les moyens de déclenchement sont disposés au moins partiellement dans la partie de base (18).

12. Dispositif selon l'une des revendications 4 à 11, comprenant en outre des moyens d'information (15) pour l'information visuelle et/ou acoustique d'un utilisateur qu'une mesure d'étalonnage a été effectuée.

13. Dispositif selon l'une des revendications 4 à 12, comprenant en outre des moyens de commutation pour l'activation et la désactivation des moyens de déclenchement.
